# EUROPÄISCHE PATENTSCHRIFT

(11) **EP 2 932 984 B1**
(45) Veröffentlichungstag und Bekanntmachung des Hinweises auf die Patenterteilung: **16.11.2016**
(21) Anmeldenummer: 15162217.2
(22) Anmeldetag: 01.04.2015
(51) Int. Cl.: A61L 2/025, H02J 5/00

(54) **VERFAHREN UND ANORDNUNG ZUR ENERGIEÜBERTRAGUNG IN EINEN SPÜLRAUM EINES REINIGUNGSAUTOMATEN**
METHOD AND ASSEMBLY FOR TRANSMISSION OF ENERGY IN A WASHING COMPARTMENT OF AN AUTOMATIC CLEANING DEVICE
PROCEDE ET SYSTEME DE TRANSFERT D'ENERGIE DANS UN ESPACE DE RINÇAGE D'UN AUTOMATE DE NETTOYAGE

(30) Priorität: 02.04.2014 DE 102014104622
(43) Veröffentlichungstag der Anmeldung: 21.10.2015
(73) Patentinhaber: Miele & Cie. KG, 33332 Gütersloh (DE)
(72) Erfinder: Malec, Tobias, 33739 Bielefeld (DE); Ernst, Holger, 33613 Bielefeld (DE)

(56) Entgegenhaltungen:
- EP-A1- 0 678 275
- WO-A1-2009/085292
- CH-A5- 696 020
- DE-A1-102006 007 169

## Beschreibung

Die Erfindung betrifft ein Verfahren und eine Anordnung zur Übertragung von elektrischer Energie in einen Spülraum eines Reinigungsautomaten, insbesondere eines Reinigungsautomaten für medizinische Instrumente.

In Krankenhäusern oder Arztpraxen werden medizinische Instrumente, die zu Operationszwecken verwendet wurden, nach der Benutzung aufbereitet. Die Aufbereitung umfasst hierbei eine Reinigung, Desinfektion, Trocknung und ggf. eine sich anschließende Sterilisation. Die Schritte des Reinigen, Desinfizieren und Trocknen werden üblicherweise in einem Reinigungs- und Desinfektionsautomaten, abgekürzt auch als Reinigungsautomat bezeichnet, durchgeführt. Dabei haben sich mit Geschirrspülmaschinen vergleichbar arbeitende Reinigungsautomaten mit mindestens einem Sprüharm, über den eine Reinigungsflüssigkeit im Spülraum versprüht wird, etabliert. Aufgebaut sind die Reinigungsautomaten häufig in Form von Schleusen, so dass Instrumente aus einem nicht desinfizierten Bereich durch den Reinigungsautomaten als Schleuse in einen desinfizierten Bereich transferiert werden.

Aufgrund von teilweise sehr hartnäckigen Verschmutzungen können manche der medizinischen Instrumente in einem derartigen Reinigungsautomaten nicht ausreichend gereinigt werden. Bei diesen Instrumenten erfolgt meist eine manuelle Vorreinigung, um die gröbsten und hartnäckigsten Verunreinigungen vorab zu lösen. Alternativ wäre es denkbar, die Vorreinigung in einem eigens dafür vorgesehenen Modul innerhalb des Spülraums vorzunehmen, wodurch die zusätzliche Arbeitszeit für die manuelle Reinigung eingespart werden könnte. Da das entsprechende Modul jedoch mit elektrischer Energie versorgt werden müsste, stellt sich das Problem, elektrische Energie in den Spülraum zu übertragen. Dabei ist zu berücksichtigen, dass im Spülraum Reinigungsflüssigkeit versprüht wird und Temperaturen bis zu etwa 120 °C (Celsius) herrschen.

Die EP 0678275 A1 beschreibt eine Geschirrspülmaschine mit einer Dosiervorrichtung und Steuermitteln, von denen ein Teil der Dosiervorrichtung zugeordnet ist und ein anderer Teil außerhalb des Arbeitsbehälters der Geschirrspülmaschine angeordnet ist. Die beiden Teile der Steuermittel können induktiv mittels eines geteilten Wechselstromüberträgers miteinander gekoppelt sein, um Antriebs- und Steuersignale für das Dosiergerät zu übertragen.

Die DE 102006007169 A1 und die CH 696020 A5 beschreiben jeweils Haushaltsgeräte, wobei zur induktiven Leistungsübertragung von dem Haushaltsgerät an eine außen an das Haushaltsgerät angesetzte Bedieneinrichtung ein induktiver Generator vorgesehen ist mit einer primären Generatorspule in dem Haushaltsgerät und einer sekundären Generatorspule in der Bedieneinrichtung.Es ist eine Aufgabe der vorliegenden Erfindung, ein Verfahren zur Übertragung von elektrischer Energie in einen Spülraum eines Reinigungsautomaten zur Versorgung eines Stromverbrauchers zu schaffen, das trotz der im Spülraum herrschenden Bedingungen sicher und zuverlässig durchgeführt werden kann. Es ist eine weitere Aufgabe, eine Anordnung zu beschreiben, mit der das Verfahren ohne großen Materialeinsatz durchgeführt werden kann.

Diese Aufgabe wird durch ein Verfahren und eine Anordnung mit den jeweiligen Merkmalen der unabhängigen Patentansprüche gelöst. Vorteilhafte Ausgestaltungen und Weiterbildungen sind in den abhängigen Ansprüchen angegeben.

Ein erfindungsgemäßes Verfahren zur Übertragung von elektrischer Energie in einen Spülraum eines Reinigungsautomaten zeichnet sich dadurch aus, dass eine mit einem im Spülraum angeordneten Stromverbraucher gekoppelte Sekundärspule in einen Wirkungsbereich einer ortsfest an einer Wandung des Spülraums angeordneten Primärspule gebracht wird und Energie induktiv von der Primärspule auf die Sekundärspule übertragen wird. Die induktive Energieübertragung ist kontaktlos. Gegenüber einer Energieübertragung mit Kontakten kann ein Schutz vor eindringendem Wasser bzw. eindringender Reinigungsflüssigkeit bei einer kontaktlosen Energieübertragung konstruktiv einfach innerhalb des Spülraums umgesetzt werden. Zudem besteht bei der kontaktlosen Energieübertragung keine Gefahr von Kontaktkorrosion, die einer sicheren und zuverlässigen Energieübertragung entgegenstehen würde und die gerade in der feuchten und warmen Atmosphäre im Spülraum andernfalls nur schwer zu verhindern wäre.

Dabei wird die Primärspule mit einer Wechselspannung in einem Frequenzbereich zwischen 20 kHz und 200 kHz, insbesondere zwischen 20 kHz und 40 kHz beaufschlagt. In diesem Frequenzbereich kann eine effektive Energieübertragung mit relativ kleinen Spulen erfolgen. Die in der Sekundärspule induzierte Wechselspannung wird dem Stromverbraucher unmittelbar zugeführt. Dadurch wird so weit wie möglich auf Elektronikkomponenten innerhalb des Spülraums verzichtet, was die Zuverlässigkeit und die Lebenserwartung der Anordnung erhöht. Die in der Sekundärspule induzierte Wechselspannung wird mindestens einem Ultraschallwandler als Stromverbraucher zugeführt. Das beschriebene
Energieübertagungsverfahren eignet sich in besonderem Maße für zur Stromversorgung von Ultraschallwandlern, die innerhalb des Spülraums angeordnet sind, da diese ohne weiteres unmittelbar mit der in der Sekundärspule induzierten Wechselspannung betrieben werden können, wenn die Primärspule entsprechend mit Wechselspannung einer Frequenz im Ultraschallbereich beaufschlagt wird.

Eine erfindungsgemäße Anordnung zur Übertragung von elektrischer Energie in einen Spülraum eines Reinigungsautomaten weist eine im Spülraum ortsfeste Primärspule und eine in den Wirkungsbereich der Primärspule einbringbare Sekundärspule auf. Dabei ist die Sekundärspule unmittelbar mit einem Stromverbraucher im Spülraum verbunden. Die Primärspule und die Sekundärspule sind zur Übertragung einer Wechselspannung in einem Frequenzbereich zwischen 20 kHz und 200 kHz, insbesondere zwischen 20 kHz und 40 kHz ausgelegt. Die Anordnung ist für mindestens einen Ultraschallwandler als Stromverbraucher ausgelegt, beispielsweise indem die Sekundärspule und/oder die Primärspule im Hinblick auf ihre Impedanz, ihre Windungszahl und ihr Windungszahlenverhältnis auf den mindestens einen Ultraschallwandler abgestimmt ist.

Es ergeben sich jeweils die im Zusammenhang mit dem Verfahren genannten Vorteile.

In einer weiteren vorteilhaften Ausgestaltung der Anordnung die Primärspule und/oder die Sekundärspule als eine Spiralspule ausgebildet. Es wird so eine besonders flache Bauweise erreicht, die gut im Bereich einer Seitenwand des Spülraums angeordnet werden kann.

In einer weiteren vorteilhaften Ausgestaltung der Anordnung ist die Primärspule in einem einstückigen Gehäuse angeordnet, das mittels eines Gewindestücks und einer Überwurfmutter in einer Montageöffnung einer Wandung des Spülraums festlegbar ist. Es wird so auf einfache Weise eine sprühwasserdichte Befestigung für die Primärspule geschaffen. Bevorzugt ist in dem Gehäuse auf einer Seite der Primärspule eine Ferritabschirmung angeordnet. Durch die Ferritabschirmung, beispielsweise in Form von flachen Ferritsegmenten, wird eine Induktion von energieverbrauchenden Wirbelströmen in der Wandung des Spülraums verhindert.

Die Erfindung wird nachfolgend anhand eines Ausführungsbeispiels mithilfe von Figuren näher erläutert. Die Figuren zeigen:
- Figur 1: eine perspektivische teilgeschnittene Ansicht eines Ausführungsbeispiels eines Reinigungsautomaten mit einer Anordnung zur Energieübertragung zu einem eingesetzten Modul;
- Figur 2: eine ebenfalls perspektivische und teilgeschnittene Ansicht des Moduls aus Figur 1;
- Figur 3: eine schematische Darstellung des Ausführungsbeispiels aus Figur 1; und
- Figur 4: eine Schnittzeichnung eines Teils einer Anordnung zur Energieübertragung in einem weiteren Ausführungsbeispiel.

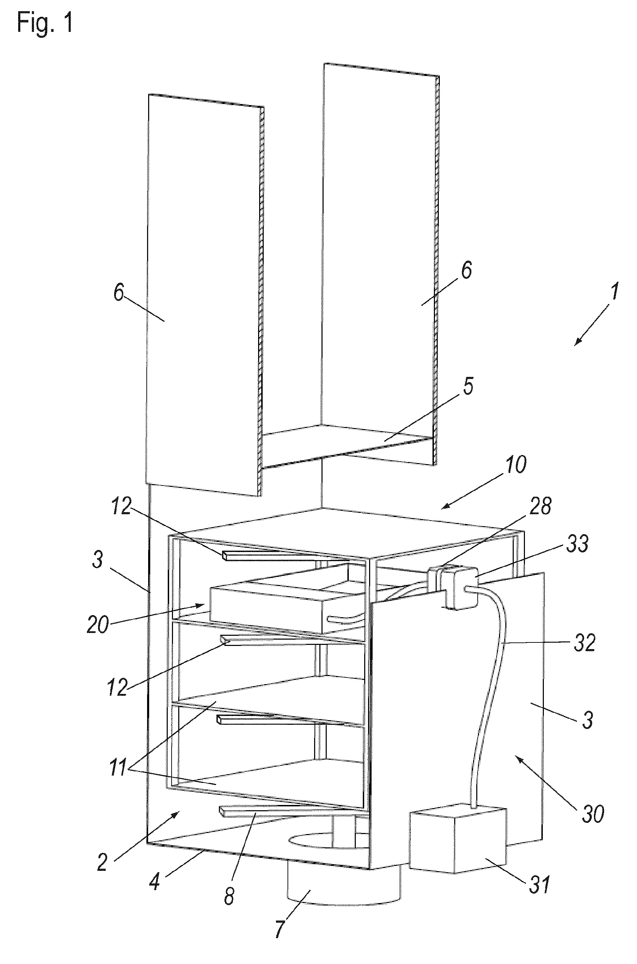

Figur 1 zeigt in einer perspektivischen teilgeschnittenen Darstellung einen Reinigungsautomat 1 zur Reinigung medizinischer Instrumente. Der Reinigungsautomat 1 weist einen Spülraum 2 auf, der durch Seitenwände 3, einen Boden 4 und eine Decke 5 begrenzt ist. Nach vorne und hinten sind Türen 6 vorgesehen, in diesem Ausführungsbeispiel ausgestaltet als vertikal verfahrbare Türen. Die Anordnung von zwei sich gegenüberliegenden Türen 6 ermöglicht es, den Reinigungsautomaten 1 in Art einer Schleuse zwischen einem nicht desinfizierten Bereich und einem desinfizierten Bereich einer Arztpraxis bzw. eines Operationsbereichs einzubauen.

Im Boden 4 des Reinigungsautomaten 1 ist ein Spültopf 7 angeordnet, in dem sich im Reinigungsautomaten 1 zirkulierende Reinigungsflüssigkeit, auch Spülflotte genannt, sammelt. Die Reinigungsflüssigkeit wird aus dem Spültopf 7 nach Filterung und ggf. weiterer Reinigung durch eine Pumpe über einen Sprüharm 8 in den Spülraum 2 zur Reinigung von eingebrachtem Spülgut gesprüht. Beispielhaft ist in der Figur 1 nur ein fest im Spülraum 2 angeordneter Sprüharm 8 dargestellt. Ein weiterer derartiger Sprüharm kann sich beispielsweise unterhalb der Decke 5 befinden.

Zur Aufnahme des Spülguts im Reinigungsautomaten 1 ist ein Spülgutwagen 10 vorgesehen, in den Spülgut auf mehreren Ebenen 11 eingeräumt werden kann. Innerhalb des Spülgutwagens 10 sind in den einzelnen Ebenen 11 weitere Sprüharme 12 angeordnet, in die ebenfalls Reinigungsflüssigkeit aus der Spülflotte des Reinigungsautomaten 1 gepumpt wird. Der übersichtlicheren Darstellung halber sind die Ebenen 11 geschlossen dargestellt. In einer Umsetzung des Reinigungsautomaten 1 bestehen die Ebenen 11 beispielsweise aus einem Drahtgittermaterial, so dass Reinigungsflüssigkeit von allen Seiten an das Spülgut gelangen kann. Zur Verbindung der weiteren Sprüharme 12 mit dem Pumpensystem des Reinigungsautomaten ist eine hier nicht dargestellte hydraulische Kupplung vorgesehen.

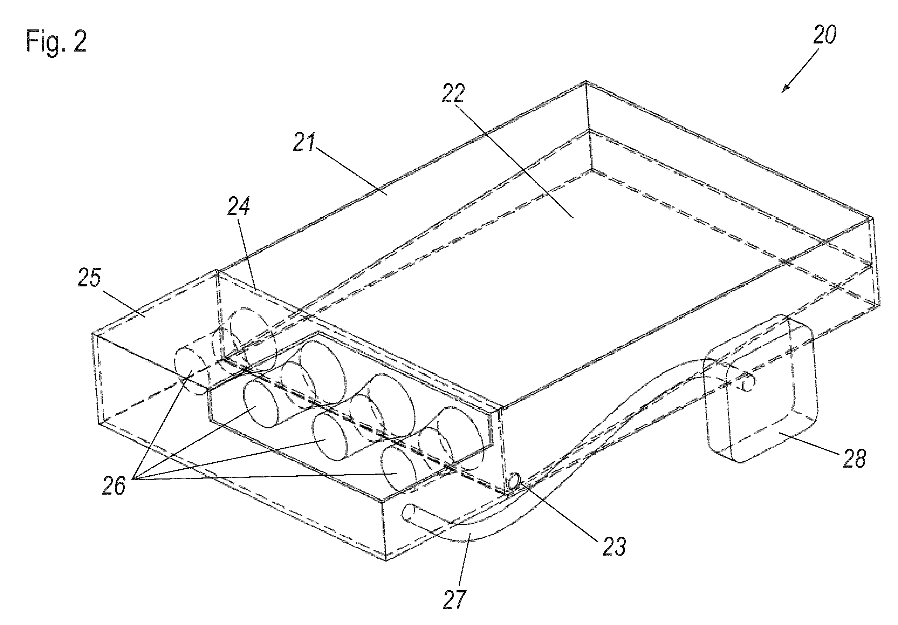

Der Reinigungsautomat 1 ist zur Aufnahme eines Ultraschallmoduls 20 vorbereitet, das im Spülraum 2 eingesetzt werden kann und mit dem besonders hartnäckig verschmutzte Instrumente zusätzlichen und besonders intensiv gereinigt werden können. Das Ultraschallmodul 20 kann in einer oder mehreren der Ebenen 11 des Spülgutwagens 10 eingesetzt werden. Die Reinigungswirkung wird durch Einstrahlen von Ultraschallwellen auf die Instrumente erzielt, wofür elektrische Energie benötigt wird, die von einer anmeldungsgemäßen Anordnung zur Übertragung von elektrischer Energie in den Spülraum 2 übertragen wird. Dieses ist nachfolgend im Zusammenhang mit den Figuren 2 bis 4 detaillierter dargestellt.

Das Ultraschallmodul 20 ist in der Figur 2 in einer ebenfalls perspektivischen und teilgeschnittenen Ansicht detaillierter dargestellt. Das Ultraschallmodul 20 umfasst eine Ultraschallwanne 21, die nach oben offen ist und nach unten eine Ablagefläche 22 für das Spülgut aufweist. Die Ablagefläche 22 kann strukturiert sein, um aufgelegten und zu reinigenden Instrumenten einen Seitenhalt zu geben. Es kann auch vorgesehen sein, dass die zu reinigenden Instrumente in hier nicht dargestellten Halterungen eingesetzt werden, um die Ablagefläche 22 selbst bzw. die Ränder der Ultraschallwanne 21 nicht zu berühren. Bei empfindlicherem Spülgut dient dieses einem Schutz vor mechanischer Beschädigung.

Die Ultraschallwanne 21 weist einen Ablauf 23 auf, der im dargestellten Beispiel aus einer oder mehreren seitlichen Bohrungen besteht. Im Betrieb des Reinigungsautomaten 1 wird Reinigungsflüssigkeit über den oder die Sprüharme 8 bzw. die weiteren Sprüharme 12 im Spülraum 2 verteilt und gelangt von oben in die Ultraschallwanne 21. Der Durchmesser des Ablaufs 23 ist so dimensioniert, dass erst bei einem höheren Stand der Reinigungsflüssigkeit in der Ultraschallwanne 21 ein Gleichgewicht zwischen zulaufender Menge an Reinigungsflüssigkeit und der durch die Bohrung 23 ablaufenden Menge an Reinigungsflüssigkeit besteht. Auf diese Weise wird erreicht, dass im Betrieb des Reinigungsautomaten 1 zumindest in bestimmten Spülphasen des Reinigungsautomaten 1 das in die Ultraschallwanne 21 eingelegte Spülgut zumindest teilweise und bevorzugt vollständig von Reinigungsflüssigkeit umgeben ist. In einem derartigen Betriebszustand des Reinigungsautomaten 1 kann eine Ultraschallreinigung des Spülguts in der Ultraschallwanne 21 erfolgen.

In einer alternativen Ausgestaltung des Ultraschallmoduls 20 kann der Ablauf 23 mit einem elektrisch ansteuerbaren Ventil versehen sein. In dem Fall kann das Auffüllen der Ultraschallwanne 21 mit der Reinigungsflüssigkeit bzw. das Ablassen der Reinigungsflüssigkeit gezielt gesteuert werden. Es ist auch möglich, ein thermisch gesteuertes Ventil zu verwenden, das aufgrund der unterschiedlichen Temperaturen während des Reinigungsvorgangs den Ablauf 23 geeignet öffnet bzw. verschließt.

Zur Durchführung der Ultraschallreinigung weist das Ultraschallmodul 20 Ultraschallwandler 26 auf, die in einer geschlossenen Umhausung 25 benachbart zur Ultraschallwanne 21 angeordnet sind. Die Ultraschallwandler 26 wirken unmittelbar auf eine Trennwand 24 ein, die eine Seitenwand der Ultraschallwanne 21 ist und die Ultraschallwanne 21 von der Umhausung 25 der Ultraschallwandler 26 trennt. Im dargestellten Beispiel sind vier nebeneinander angeordnete Ultraschallwandler 26 vorgesehen. Diese Ultraschallwandler 26 sind beispielsweise piezoelektrisch arbeitende Wandler, die Schwingungen im Ultraschallbereich, beispielsweise zwischen 20 Kilohertz (kHz) und 200 kHz und insbesondere zwischen 20 kHz und 40 kHz, auf die Trennwand 24 und damit über die Reinigungsflüssigkeit auf das in der Ultraschallwanne 21 angeordnete Spülgut übertragen.

Die Ultraschallwandler 26 sind über ein elektrisches Kabel 27 mit einer Sekundärspule 281 verbunden, die in einem Kupplungselement 28 angeordnet ist.

Die weiteren zum Betrieb der Ultraschallwandler 26 benötigten Komponenten sind in der Figur 1 dargestellt. An einer der Seitenwände 3 des Reinigungsautomaten 1 ist eine Ultraschallgeneratoreinheit 30 angeordnet, die einen Ultraschallsignalgenerator 31 aufweist, der über ein Kabel 32 mit einem Kupplungsgegenstück 33 verbunden ist. Im Betrieb des Ultraschallmoduls 20 wird vom Ultraschallsignalgenerator 31 ein elektrisches Wechselspannungssignal im genannten Frequenzbereich von 20 kHz bis etwa 200 kHz erzeugt, das über das Kabel 32 auf eine in dem Kupplungsgegenstück 33 angeordnete Primärspule 331 übertragen wird. Das Kabel 32 ist dabei ebenso wie das Kabel 27 bevorzugt abgeschirmt, so dass keine Störstrahlung abgegeben wird.

Die Primärspule 331 des Kupplungsgegenstücks 33 überträgt vom Ultraschallsignalgenerator 31 bereitgestellte elektrische Energie induktiv auf die Sekundärspule 281 des Kupplungselements 28 des Ultraschallmoduls 20. Dabei sind das Kupplungselement 28 und das Kupplungsgegenstück 33 abgedichtet, so dass die Sekundär- bzw. Primärspulen 281 und 331 vor der verspritzen Reinigungsflüssigkeit im Spülraum 2 geschützt sind. Das Kupplungsgegenstück 33 ist zudem wasserdicht in der Seitenwand 3 angeordnet.

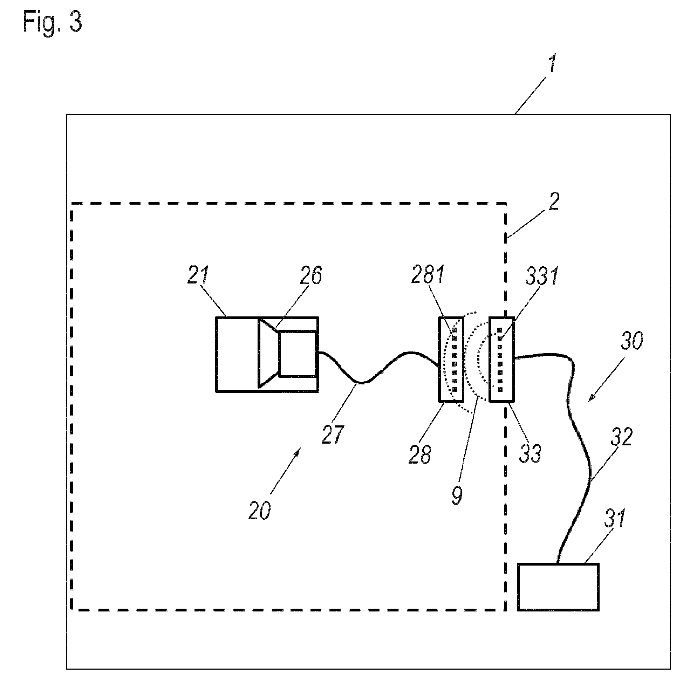

Die Anordnung zur Übertragung der elektrischen Energie in den Spülraum 2, umfassend die Primärspule 331 und die Sekundärspule 281, ist in der Figur 3 nochmals schematisch dargestellt. Dabei ist ein magnetisches Wechselfeld 9, über das die Energie übertragen wird, angedeutet.

Die Energieübertragung von der Primärspule 331 und die Sekundärspule 281 erfolgt in dem dargestellten Ausführungsbeispiel bei der Frequenz, bei der auch die Ultraschallwandler 26 betrieben werden. Die Ultraschallwandlern 26 als Stromverbraucher können folglich unmittelbar mit der Sekundärspule 281 verbunden werden. Dadurch kann die Ultraschallgeneratoreinheit 30 außerhalb des Spülraums 2 angeordnet werden und es werden im Spülraum 2 selbst keine weiteren elektronischen Komponenten benötigt, was mehrere Vorteile hat. Zum einen herrschen im Spülraum 2 hohe Temperaturen, die in der Desinfektionsphase bis auf ca. 95°C und in der Trocknungsphase bis auf ca. 120°C steigen können. Derartig hohe Temperaturen sind für elektronische Komponenten unter Umständen problematisch und können zu einer Verkürzung ihrer Lebensdauer führen. Zudem erweist es sich im praktischen Betrieb des Reinigungsautomaten 1 als vorteilhaft, wenn mehrere Ultraschallmodule 20 vorhanden sind, wodurch beispielsweise ein Ultraschallmodul 20 beladen werden kann, während ein weiteres Ultraschallmodul 20 sich im Reinigungsautomaten 1 befindet. Die Anordnung der Ultraschallgeneratoreinheit 30 am Reinigungsautomaten 1 führt in diesem Fall zu einer kostengünstigen Ausgestaltung der Ultraschallmodule 20, da diese nur die Ultraschallwandler 26 und nicht die gesamten elektronischen Komponenten aufweisen müssen.

Es kann vorgesehen sein, dass nach Einschieben des Spülgutwagens 10 mit dem eingesetzten Ultraschallmodul 20 das Kupplungselement 28 händisch auf das Kupplungsgegenstück 33 aufgesteckt wird und mit diesem verbunden und ggf. verriegelt wird. Diese Verbindung bzw. Verriegelung wird dann ebenfalls händisch gelöst, bevor der Spülgutwagen zur anderen Seite des Reinigungsautomaten 1 nach erfolgter Reinigung entnommen wird. Es ist alternativ denkbar, dass das Kupplungselement 28 in eine Halterung am Spülgutwagen 10 eingesetzt wird, wobei diese Halterung so angeordnet ist, dass beim Einschieben des Spülgutwagens 10 das Kupplungselement 28 geeignet vor dem Kupplungsgegenstück 33 positioniert ist, so dass die induktive Energieübertragung erfolgen kann.

Neben der speziellen Ultraschallbehandlung erfahren in das Ultraschallmodul 20 eingelegte Instrumente auch die normalen Reinigungs-, Desinfektions- und Trocknungsschritte des Reinigungsautomaten 1. Es findet insofern eine vollständige Integration der Ultraschallreinigung in den sonstigen Reinigungsprozess statt.

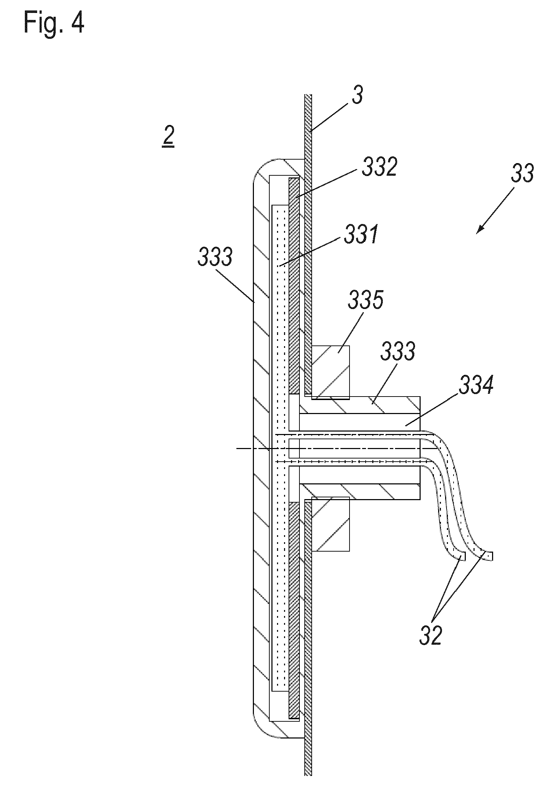

Figur 4 zeigt eine Schnittzeichnung eines Ausführungsbeispiels eines Kupplungsgegenstücks 33 für eine Anordnung zur Übertragung von elektrischer Energie in einen Spülraum 2 eines Reinigungsautomaten. Das Kupplungsgegenstück 33 ist durch eine Öffnung in einer Seitenwand 3 eines Reinigungsautomaten eingesetzt, beispielsweise des in Figur 1 dargestellten Reinigungsautomaten 1. Zur Montage des Kupplungsgegenstücks 33 der Anordnung zur Energieübertragung wird lediglich eine in diesem Fall kreisrunde Montageöffnung in einer Wandung benötigt, hier in einer Seitenwand 3, die den Spülraum 2 umgibt. In der Figur 4 befindet sich der Spülraum 2 auf der linken Seite der Seitenwand 3.

Das Kupplungsgegenstück 33 weist eine Primärspule 331 auf, die in dem dargestellten Ausführungsbeispiel spiralförmig gewickelt ist. Mit einer derartigen Spiralspule kann ein möglichst flacher Aufbau des Kupplungsgegenstücks 33 erreicht werden. In Richtung der Seitenwand 3, also in der Figur 4 von der Primärspule 331 aus gesehen rechts, sind Ferritsegmente 332 zur Abschirmung eines magnetischen Felds der Spule 331 angeordnet. Diese so gebildete Ferritabschirmung verhindert ein Eindringen magnetischer Feldlinien in die üblicherweise metallische Seitenwand 3, in der anderenfalls verlustreiche Wirbelströme induziert würden. Für eine besonders effektive Abschirmung ragen die Ferritsegmente 332 radial über den Radius der Primärspule 331 hinaus.

Die Anordnung aus Primärspule 331 und Ferritsegmenten 332 ist in einem einstückigen Gehäuse 333 angeordnet. Das Gehäuse 333 umschließt die Primärspule 331 und die Ferritsegmente 332 im Bereich des Spülraums 2 vollständig. Das Gehäuse 333 geht zur gegenüberliegenden Seite hin in ein Gewindestück über, das durch die zuvor genannte Öffnung in der Seitenwand 3 gesteckt ist. Zentral im Gewindestück ist eine Kabeldurchführung 334 eingesetzt, durch die Anschlusskabel 32 für die Primärspule 331 geführt sind. Außen ist das Gewindestück des Gehäuses 333 mit einem Gewinde versehen, auf das eine Überwurfmutter 335 geschraubt ist, mit der das Gehäuse 333 und damit das gesamte Kupplungsgegenstück 33 an der Seitenwand 3 festgelegt ist. Dabei kann an dem Gehäuse 333 in den im Spülraum 2 liegenden und an der Seitenwand 3 anliegenden Bereich eine Nut zur Aufnahme eines O-Rings eingebracht sein, mit dem das Gehäuse 333 im Bereich der Überwurfmutter 335 gegenüber der Seitenwand 3 abgedichtet ist, um ein Austreten von Feuchtigkeit aus dem Spülraum 2 durch die Montageöffnung in der Seitenwand 3 zu verhindern.

Das Gehäuse 333 ist bevorzugt aus einem hitzefesten und gegenüber der verwendeten Reinigungsflüssigkeit resistenten Kunststoffmaterial gefertigt. Es kann zunächst in zwei Teilen produziert werden, die nach Einsetzen der Primärspule 331 und der Ferritsegmente 332 sowie ggf. der Kabeldurchführung 334 bevorzugt untrennbar miteinander verbunden, beispielsweise verschweißt, insbesondere plasmaverschweißt, sind.

In dem dargestellten Ausführungsbeispiel weist das Kupplungsgegenstück 33 einen bestmöglichen Schutz gegen eindringendes Wasser und einen sehr flachen Aufbau auf. Zudem ist eine einfache Montage in den Reinigungsautomaten 1 gegeben.

### Bezugszeichenliste

- 1: Reinigungsautomat
- 2: Spülraum
- 3: Seitenwand
- 4: Boden
- 5: Decke
- 6: Tür
- 7: Spültopf
- 8: Sprüharm
- 9: magnetisches Wechselfeld

- 10: Spülgutwagen
- 11: Ebene
- 12: Sprüharm

- 20: Ultraschallmodul
- 21: Ultraschallwanne
- 22: Ablagefläche
- 23: Ablauf
- 24: Trennwand
- 25: Umhausung
- 26: Ultraschallwandler
- 27: Kabel
- 28: Kupplungselement
- 281: Sekundärspule

- 30: Ultraschallgeneratoreinheit
- 31: Ultraschallsignalgenerator
- 32: Kabel
- 33: Kupplungsgegenstück
- 331: Primärspule
- 332: Ferritsegment
- 333: Gehäuse
- 334: Kabeldurchführung
- 335: Überwurfmutter

## Patentansprüche

1. Verfahren zur Übertragung von elektrischer Energie in einen Spülraum (2) eines Reinigungsautomaten (1), wobei
i. eine mit einem sich im Spülraum angeordneten Stromverbraucher gekoppelte Sekundärspule (281) in den Wirkungsbereich einer ortsfest an einer Wandung des Spülraums (2) angeordneten Primärspule (331) gebracht wird und
ii. Energie induktiv von der Primärspule (331) auf die Sekundärspule (281) übertragen wird,
**dadurch gekennzeichnet, dass**
die Primärspule (331) mit einer Wechselspannung in einem Frequenzbereich zwischen 20 kHz und 200 kHz beaufschlagt wird,
und dass die in der Sekundärspule (281) induzierte Wechselspannung mindestens einem Ultraschallwandler (26) als Stromverbraucher unmittelbar zugeführt wird.

2. Anordnung zur Übertragung von elektrischer Energie in einen Spülraum (2) eines Reinigungsautomaten (1), wobei die Anordnung eine im Spülraum (2) ortsfeste Primärspule (331) und eine in den Wirkungsbereich der Primärspule (331) einbringbare Sekundärspule (281) aufweist, wobei die Sekundärspule (281) unmittelbar mit einem Stromverbraucher im Spülraum (2) verbunden ist,
**dadurch gekennzeichnet, dass**
die Primärspule (331) und die Sekundärspule (281) zur Übertragung einer Wechselspannung in einem Frequenzbereich zwischen 20 kHz und 200 kHz ausgelegt sind,
und dass der Stromverbraucher mindestens ein Ultraschallwandler (26) ist.

3. Anordnung nach Anspruch 2, bei der die Primärspule (331) und/oder die Sekundärspule (281) als eine Spiralspule ausgebildet ist.

4. Anordnung nach einem der Ansprüche 2 bis 3, bei der die Primärspule (331) in einem einstückigen Gehäuse (333) angeordnet, das mittels eines Gewindestücks und einer Überwurfmutter (334) in einer Montageöffnung einer Wandung des Spülraums (2) festlegbar ist.

5. Anordnung nach Anspruch 4, bei der in dem Gehäuse (333) auf einer Seite der Primärspule (331) eine Ferritabschirmung angeordnet ist.

## Claims

1. Method for transmitting electrical energy in a washing chamber (2) of a cleaning machine (1),
i. a secondary coil (281) that is coupled to a current consumer arranged in the washing chamber being brought into the active region of a primary coil (331) that is arranged in a stationary manner on a wall of the washing chamber (2), and
ii. energy being transmitted by induction from the primary coil (331) to the secondary coil (281),
**characterised in that**
an alternating voltage in a frequency range of between 20 kHz and 200 kHz is applied to the primary coil (331),
and **in that** the alternating voltage induced in the secondary coil (281) is directly supplied to at least one ultrasonic transformer (26) as a current consumer.

2. Arrangement for transmitting electrical energy in a washing chamber (2) of a cleaning machine (1), the arrangement comprising a stationary primary coil (331) that is stationary in the washing chamber (2), and a secondary coil (281) that can be brought into the active region of the primary coil (331), the secondary coil (281) being directly connected to a current consumer in the washing chamber (2),
**characterised in that**
the primary coil (331) and the secondary coil (281) are designed to transmit an alternating voltage in a frequency range of between 20 kHz and 200 kHz, and **in that** the current consumer is at least one ultrasonic transformer (26).

3. Arrangement according to claim 2, in which the primary coil (331) and/or the secondary coil (281) is designed as a spiral coil.

4. Arrangement according to either claim 2 or claim 3, in which the primary coil (331) is arranged in an integral housing (333) that can be fixed in a mounting opening of a wall of the washing chamber (2) by means of a threaded piece and a cap nut (334).

5. Arrangement according to claim 4, in which a ferrite shielding is arranged in the housing (333) on one side of the primary coil (331).

## Revendications

1. Procédé de transmission d'énergie électrique dans un espace de lavage (2) d'un appareil de nettoyage automatique (1), dans lequel
i. une bobine secondaire (281) couplée à un consommateur de courant disposé dans l'espace de lavage est mise en place dans le champ d'action d'une bobine primaire (331) disposée de façon fixe sur une paroi de l'espace de lavage (2), et
ii. de l'énergie est transmise par induction à partir de la bobine primaire (331) vers la bobine secondaire (281),
**caractérisé en ce que**
la bobine primaire (331) est alimentée avec une tension alternative dans une plage de fréquences entre 20 kHz et 200 kHz,
et **en ce que** la tension alternative induite dans la bobine secondaire (281) est conduite directement à un transducteur ultrasonique (26) au moins au nombre de un en tant que consommateur de courant.

2. Ensemble de transmission d'énergie électrique dans un espace de lavage (2) d'un appareil de nettoyage automatique (1), dans lequel l'ensemble présente une bobine primaire (331) fixe dans l'espace de lavage (2) et une bobine secondaire (281) pouvant être introduite dans le champ d'action de la bobine primaire (331), dans lequel la bobine secondaire (281) est raccordée directement à un consommateur de courant dans l'espace de lavage (2),
**caractérisé en ce que**
la bobine primaire (331) et la bobine secondaire (281) sont conçues pour la transmission d'une tension alternative dans une plage de fréquences entre 20 kHz et 200 kHz,
et **en ce que** le consommateur de courant est au moins un transducteur ultrasonique (26).

3. Ensemble selon la revendication 2, dans lequel la bobine primaire (331) et/ou la bobine secondaire (281) est constituée en tant que bobine en spirale.

4. Ensemble selon l'une des revendications 2 à 3, dans lequel la bobine primaire (331) est disposée dans un boîtier (333) d'une seule pièce qui peut être immobilisé dans une ouverture de montage d'une paroi de l'espace de lavage (2) au moyen d'une pièce filetée et d'un écrou-raccord (334).

5. Ensemble selon la revendication 4, dans lequel un blindage en ferrite est disposé dans le boîtier (333) sur un côté de la bobine primaire (331).
